(19) Europäisches Patentamt
European Patent Office
Office européen des brevets

(11) **EP 3 214 562 B1**

(12) **EUROPÄISCHE PATENTSCHRIFT**

(45) Veröffentlichungstag und Bekanntmachung des Hinweises auf die Patenterteilung:
**14.11.2018 Patentblatt 2018/46**

(51) Int Cl.:
*G16H 30/20* *(2018.01)*   *G16H 40/63* *(2018.01)*

(21) Anmeldenummer: **17174931.0**

(22) Anmeldetag: **08.06.2017**

(54) **BESTIMMEN EINER BILDSERIE ABHÄNGIG VON EINER SIGNATURMENGE**

DETERMINING A SERIES OF IMAGES DEPENDING ON A SIGNATURE SET

DÉTERMINATION D'UNE SÉRIE D'IMAGES EN FONCTION D'UN ENSEMBLE DE SIGNATURES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.07.2016 DE 102016212888**

(43) Veröffentlichungstag der Anmeldung:
**06.09.2017 Patentblatt 2017/36**

(73) Patentinhaber: **Siemens Healthcare GmbH**
**91052 Erlangen (DE)**

(72) Erfinder: **Canda, Valer**
**91052 Erlangen (DE)**

(56) Entgegenhaltungen:
**US-A1- 2010 232 661    US-A1- 2013 129 198**
**US-A1- 2015 154 356    US-A1- 2015 161 147**
**US-B1- 8 094 901    US-B1- 8 976 190**

EP 3 214 562 B1

**Beschreibung**

Die vorliegende Erfindung betrifft das Bestimmen einer Bildserie aus einer Menge von Bildserien abhängig von einer Signaturmenge. Insbesondere betrifft die vorliegende Erfindung das Bestimmen von Bildern, welche von einem bildgebenden System erzeugt werden, um diese Bilder darzustellen und anhand dieser Bilder eine radiologische Auswertung durchführen zu können.

Die US 2010/0232661 A1 offenbart eine Bildsuche, bei welcher ein Bild ermittelt und dargestellt wird, welches einem Bild eines Diagnoseobjekts auf der Basis eines Merkmalsumfangs ähnlich ist, welcher von dem Bild des Diagnoseobjekts extrahiert wurde. Dazu werden Attribute eines Bildes des Diagnoseobjekts, welches in einer Gruppe von Bildern des Diagnoseobjekts enthalten ist, abgeschätzt, und es wird ein erster Merkmalsumfang des Bildes des Diagnoseobjekts berechnet, welcher dem abgeschätzten Attribut entspricht. Dann wird in einer Gruppe von Bildern ein dem Bild des Diagnoseobjekts ähnliches Bild gesucht, indem der erste Merkmalsumfang mit einem zweiten Merkmalsumfang verglichen wird, welcher ein Merkmalsumfang eines Bildes in der Gruppe ist und dem abgeschätzten Attribut entspricht.

Die US 2015/0161147 A1 offenbart ein Bestimmen, ob Bilder einen Bezug zu Videos aufweisen. Dazu werden Merkmale der Bilder mit Merkmalen der Videos verglichen.

Die US 2015/0154356 A1 adressiert das Problem, geeignete Bilder für eine effiziente klinische Bewertung auszuwählen. Dazu werden Layouts mit Platzhaltern für mehrere Segmente erzeugt. Wenn ausgewählte Bilddaten geladen werden, werden diejenigen Bilddaten dem jeweiligen Platzhalter zugewiesen, die am besten den Eigenschaften der Platzhalter entsprechen. Diese Bilddaten werden einem Benutzer dargestellt. Die ausgewählten Platzhalter können bestimmte Eigenschaften, wie z.B. einen Zeitpunkt, eine Erfassungszahl, eine Bildaufbereitungsmodalität und einen Rekonstruktionstyp, umfassen.

Beispielsweise bei der Befundung komplexer MR-Datensätze hat sich ein Workflow-basierter Ansatz als effizient erwiesen, um eine möglichst rasche Auswertung von anhand der MR-Datensätzen erzeugten MR-Bildern zu ermöglichen. Dabei werden die MR-Bilder mit Hilfe eines Layouts auf einer Anzeige dargestellt, wobei das Layout mehrere Segmente umfasst. In jedem dieser Segmente wird ein MR-Bild oder eine MR-Bildserie dargestellt.

Dabei wird in jedem Segment (sofern vorhanden) ein MR-Bild oder eine MR-Bildserie dargestellt, welche einem bestimmten Teil oder Organsystem des zu untersuchenden Patienten entspricht. Beispielsweise kann in einem Segment eine MR-Bildserie des Skelettsystems des Patienten dargestellt werden, während in anderen Segmenten eine MR-Bildserie der Thorax oder des Beckens oder eines bestimmten Organs des Patienten abgebildet ist. Eine weitere Möglichkeit ist, dass sich die Segmente hinsichtlich eines Bild-Kontrastes oder hinsichtlich einer Orientierung oder hinsichtlich einer Phasen-Eigenschaft unterscheiden. Wenn für ein Segment mehrere verschiedene Bildserien dargestellt werden können, wird diejenige Bildserie dargestellt, welche für die aktuelle Auswertung die sinnvollste ist.

Die vorliegende Erfindung stellt sich die Aufgabe, die Bestimmung der Bildserie, welche in einem bestimmten Segment des Layouts darzustellen ist, gegenüber dem Stand der Technik zu verbessern.

Erfindungsgemäß wird diese Aufgabe durch ein Verfahren zum Bestimmen von mittels eines bildgebenden Systems erzeugenden Bildern zur Auswertung durch eine Bedienperson nach dem Anspruch 1, durch ein bildgebendes System nach Anspruch 9, durch ein Computerprogrammprodukt nach Anspruch 11 und durch einen elektronisch lesbaren Datenträger nach Anspruch 12 gelöst. Die abhängigen Ansprüche definieren bevorzugte und vorteilhafte Ausführungsformen der vorliegenden Erfindung.

Es wird ein Verfahren zum Bestimmen einer Bildserie aus einer Menge von mehreren Bildserien bereitgestellt. Dabei umfasst das Verfahren unter anderem folgende Schritte:

- Erstellen jeweils einer Signatur für jede der mehreren Bildserien, indem aus einer Menge von Attributen der jeweiligen Bildserie diese Signatur gebildet wird. In diesem Schritt wird das Attribut oder in der Regel die Attribute der jeweiligen Signatur aus den Attributen der zugehörigen Bildserie gebildet. Dazu können bestimmte Attribute der Bildserie als ein Attribut der Signatur übernommen werden, Attribute der Bildserie verknüpft werden, um dadurch ein Attribut der Signatur zu bestimmen, oder ein Attribut der Signatur anderweitig aus den Attributen der Bildserie abgeleitet werden.

- Ermitteln einer Signatur aus den Signaturen der Bildserien, welche einer vorgegebenen Signaturmenge am ähnlichsten ist. In diesem Schritt wird aus der Menge der Bildserien anhand der Signaturen diejenige Signatur (und damit Bildserie) ermittelt, welche die größte Ähnlichkeit mit der vorgegebenen Signaturmenge aufweist.

Das Verfahren ermöglicht es, aus einer Menge von Bildserien diejenige Bildserie zu bestimmen, welche eine vorgegebene Bedingung am besten erfüllt. Indem das Maß für das Erfüllen der vorgegebenen Bedingung quasi in ein Ähnlichkeitsmaß zwischen einer Signatur und einer Signaturmenge, mit welcher die Bedingung beschrieben wird, übersetzt wird, bietet es ein einfaches und auf zahlreiche Bedingungen anpassbares Vorgehen zur automatischen Bestimmung der am besten passenden Bildserie.

Die vorliegende Erfindung kann eingesetzt werden, um aus einer Menge von Bildserien diejenige Bildserie zu bestimmen, welche am besten geeignet ist, um in einem bestimmten Segment eines Layouts dargestellt zu werden. Darüber hinaus existieren insbesondere folgende (auch kombinierbare) Vorgehen, bei welchen die vorliegende Erfindung eingesetzt werden kann:

- Bildauswertung
  Mit der vorliegenden Erfindung kann aus einer Menge von Bildserien diejenige Bildserie ausgewählt werden, welche am besten für eine medizinische Bildauswertung geeignet ist. Dabei kann die medizinische Bildauswertung beispielsweise eine Ermittlung der Ausmaße eines Organs des Untersuchungsobjekts umfassen.

- Darstellung
  Mit der vorliegenden Erfindung kann aus einer Menge von Bildserien diejenige Bildserie ausgewählt werden, welche am besten zur Darstellung in einem Befundbericht und/oder auf einem Filmingsheet und damit zur Befundung oder zum Nachweis eines bestimmten Zustands z.B. eines Patienten geeignet ist. Dabei wird unter einem Filmingsheet bzw. Filmsheet eine Art durchsichtige Folie verstanden, auf die ein bestimmtes Bild oder mehrere bestimmte Bilder der Bildserie aufgebracht (z.B. aufgedruckt) werden, um z.B. vor einem Lichtkasten betrachtet zu werden. Ein Filmingsheet kann aber auch Bestandteil des Befundberichts sein.

- Bildnachbearbeitung
  Mit der vorliegenden Erfindung kann aus einer Menge von Bildserien diejenige Bildserie ausgewählt werden, welche am besten für eine Bildnachbearbeitung geeignet ist. Die Bildnachbearbeitung umfasst beispielsweise die Berechnung einer Perfusionskarte, die Berechnung von Subtraktionsserien oder die Durchführung einer Bewegungskorrektur für die ausgewählte(n) Bildserie(n). Im Allgemeinen umfasst die Bildnachbearbeitung die Berechnung einer oder mehrerer neuen Bildserien ausgehend von einer oder mehreren ursprünglichen Bildserien.

- Weiterleitung
  Mit der vorliegenden Erfindung kann aus einer Menge von Bildserien diejenige Bildserie ausgewählt werden, welche am besten für eine automatische Weiterleitung beispielsweise an andere Systeme (z.B. PACS ("Picture Archiving and Communication System")) geeignet ist. Beispielsweise kann diejenige Bildserie zur Weiterleitung ermittelt werden, welche am besten einen bestimmten Bereich des Untersuchungsobjekts abbildet, auf welchen das Empfangssystem (oder die Bedienpersonen des Empfangssystems) spezialisiert sind.

- Archivierung
  Mit der vorliegenden Erfindung kann aus einer Menge von Bildserien diejenige Bildserie ausgewählt werden, welche am besten für eine automatische Archivierung geeignet ist. Beispielsweise kann erfindungsgemäß genau diejenige Bildserie archiviert werden, welche für den Nachweis einer bestimmten Befundung am besten geeignet ist.

Dabei umfasst das Verfahren folgende weitere Schritte:

- Erzeugen eines Layouts mit mehreren Segmenten, um dieses Layout auf einer Anzeige darzustellen. Das darzustellender Layout oder der Anzeigeinhalt unterteilt sich demnach in mehrere Segmente.

- Zuweisen einer jeweiligen Signaturmenge oder Signatursammlung an jedes Segment. In diesem Schritt wird bzw. werden jedem Segment eine oder mehrere Signaturen zugewiesen, welche in einer jeweiligen Signaturmenge zusammengefasst sind. Dabei wird jede Signatur aus einer Menge von mindestens einem Attribut (in der Regel aber mehreren Attributen) einer Bildserie gebildet. Unter einer Bildserie wird dabei eine geordnete Menge von einem Bild oder meist mehreren Bildern verstanden. Die Signaturmenge jedes Segments kann erweitert, verkleinert oder verändert werden.

- Erstellen jeweils einer Signatur für mehrere Bildserien. In diesem Schritt wird für mehrere Bildserien, welche insbesondere mit dem bildgebenden System erstellt wurden, pro Bildserie eine Signatur erstellt.

- Ermitteln einer Signatur aus den Signaturen der Bildserien, welche der Signaturmenge des jeweiligen Segments am ähnlichsten ist. In diesem Schritt wird für jedes der Segmente eine der Signaturen der Bildserien ermittelt. Die für das jeweilige Segment ermittelte Signatur ist dabei diejenige der Signaturen der Bildserien, welche die größte Ähnlichkeit mit der Signaturmenge des jeweiligen Segments aufweist.

- Darstellen derjenigen der Bildserien in dem jeweiligen Segment, deren Signatur in dem Schritt des Ermittelns der Signatur für jedes der Segmente als ähnlichste Signatur ermittelt wurde. In diesem Schritt wird für jedes Segment des Layouts jeweils eine Bildserie dargestellt. Die pro Segment dargestellte Bildserie entspricht dabei derjenigen Bildserie, deren Signatur für das jeweilige Segment (genauer für die Signaturmenge des jeweiligen Segments) als ähnlichste Signatur ermittelt wurde.

Im Gegensatz zum Stand der Technik, nach dem pro Segment beispielsweise eine bestimmte Bezeichnung einer Bildserie oder bestimmte Bedingungen für die darzustellende Bildserie angegeben werden, muss erfindungsgemäß für jedes Segment nur die entsprechende Signaturmenge definiert werden. Eine Signatur dieser Signaturmenge kann dabei beispielsweise anhand einer vorab erzeugten Beispiel-Bildserie erstellt werden.

Die Attribute einer Signatur können beispielsweise jeweils aufweisen:

- einen Namen,

- einen Datentyp, z.B. Integer-Zahl, Floating-Point-Zahl, Datum, String, UDI (d.h. eine Sequenz von Ziffern und Punkten), binäre Sequenz, usw.

- Eine oder mehrere Daten des jeweiligen Typs. Ein Attribut kann nur einen Datenwert des jeweiligen Datentyps, also nur eine Floating-Point-Zahl oder nur einen String enthalten. Es ist aber auch möglich, dass ein Attribut beispielsweise mehrere Datenwerte, z.B. drei Floating-Point-Zahlen, umfasst, wobei diese drei Floating-Point-Zahlen als ein 3D-Vektor interpretiert werden können, welcher eine Position im Raum (z.B. Raum-Koordinaten [x, y, z] in Zentimeter) angibt.

Der vorab beschriebene Schritt des Ermittelns der ähnlichsten Signatur kann folgende Unterschritte umfassen:

- Bestimmen für alle Bildserien einer Distanz oder Entfernung zwischen der Signatur der jeweiligen Bildserie und der Signaturmenge. In diesem Unterschritt wird für jede Bildserie die Distanz zwischen ihrer Signatur und der Signaturmenge ermittelt.

- Bestimmen der ähnlichsten Signatur als diejenige Signatur, welche die geringste Distanz zu der Signaturmenge aufweist.

Indem automatisch für alle Signaturen der Bildserien eine Distanz zu der Signaturmenge eines bestimmten Segments bestimmt wird und anschließend diejenige Signatur als die ähnlichste Signatur ermittelt wird, welche die kleinste Distanz zu der Signaturmenge aufweist, kann die Bestimmung der in dem bestimmten Segment darzustellenden Bildserie sehr gut automatisiert werden.

Für die Bestimmung der Distanz zwischen einer Signatur und einer Signaturmenge existieren folgende erfindungsgemäße Möglichkeiten. Dabei wird bei jeder dieser Möglichkeiten die Distanz zwischen der Signatur und der Signaturmenge abhängig von Distanzen zwischen der Signatur und jeder Signatur der Signaturmenge bestimmt.

- Bei der ersten Möglichkeit wird die Distanz zwischen der Signatur und der Signaturmenge als diejenige Distanz bestimmt, welche die kleinste Distanz aus der Menge der Distanzen zwischen der Signatur und jeder Signatur der Signaturmenge ist.

- Bei der zweiten Möglichkeit entspricht die Distanz zwischen der Signatur und der Signaturmenge dem Mittelwert der Distanzen zwischen der Signatur und jeder Signatur der Signaturmenge.

- Bei der dritten Möglichkeit wird die Distanz zwischen der Signatur und der Signaturmenge als eine gewichtete Summe der Distanzen zwischen der Signatur und jeder Signatur der Signaturmenge bestimmt. Dabei können die Gewichte der Signaturen in der Signaturmenge abhängig von ihrer Position in der Signaturmenge verringert werden. Beispielsweise kann die Position der jeweiligen Signatur in der Signaturmenge vom Erstellungsdatum der Signatur (oder vom Zeitpunkt, zu welchem die Signatur der Signaturmenge beigefügt wurde) abhängen. Dadurch wäre gewährleistet, dass die Signatur (und damit die zugehörige Bildserie) ein umso größeres Gewicht erhält, je jünger die Signatur in der Signaturmenge ist.

Gemäß den vorab beschriebenen erfindungsgemäßen Möglichkeiten wird die Bestimmung der Distanz zwischen einer Signatur und einer Signaturmenge quasi anhand der Bestimmung mehrerer Distanzen zwischen jeweils zwei Signaturen vorgenommen. Die Distanz zwischen zwei Signaturen kann dabei abhängig von den Distanzen bestimmt werden, welche sich entsprechende Attribute der beiden Signaturen zueinander aufweisen.

Alle Signaturen weisen dieselben Attribute auf.

Bisweilen tritt der Fall auf, dass bestimmte Attribute einer Bildserie nicht gesetzt sind. Abhängig von der Art und Weise, wie ausgehend von den Attributen der Bildserie die Attribute der Signatur gebildet werden, kann dann auch ein Attribut oder können mehrere Attribute der zugehörigen Signatur nicht gesetzt sein, d.h. den Wert 0 bzw. "" oder N/A ("not available") aufweisen. Das heißt, wenn eine Signatur ein Attribut aufweist, kann der Wert dieses Attributs gesetzt oder nicht gesetzt sein.

Es sei angenommen, dass die Attribute jeder Signatur in derselben Weise sortiert sind, so dass das n-te Attribut jeder Signatur denselben Typ und dieselbe Art von Attribut beschreibt. Um nun die Distanz zwischen zwei Signaturen zu bestimmen, wird in einem ersten Schritt die Distanz zwischen den ersten Attributen der beiden Signaturen, die Distanz zwischen den zweiten Attributen der beiden Signaturen, usw. ermittelt. In einem zweiten Schritt werden die Distanz-Beiträge für die einzelnen Attribute entweder einfach addiert oder zuerst gewichtet und anschließend addiert. Mit anderen Worten entspricht die Distanz zwischen zwei Signaturen insbesondere der Summe der Distanzen der einzelnen Attribute der beiden Signaturen, wobei die Distanzen der einzelnen Attribute jeweils gewichtet werden können, wie es der folgenden Gleichung (1) zu entnehmen ist.

$$D(Sig1, Sig2) = \sum_{i=1}^{N} G_i \times DistAttr\big(Sig1.Attr(i), Sig2.Attr(i)\big) \qquad (1)$$

Dabei entspricht
D(Sig1, Sig2) der zu berechnenden Distanz zwischen den beiden Signaturen Sig1 und Sig2;
N der Anzahl der Attribute der beiden Signaturen;
DistAttr() einer Funktion, mit welcher die Distanz zwischen zwei Attributen bestimmt wird;
$G_i$ dem Gewicht, mit dem der entsprechende Funktionswert multipliziert wird;
Sig1.Attr(i) dem i-ten Attribut der ersten Signatur; und
Sig2.Attr(i) dem i-ten Attribut der zweiten Signatur.

Die Funktion DistAttr() kann dabei abhängig von dem Datentyp der beiden Attribute, zwischen denen die Distanz zu ermitteln ist, wie folgt gebildet werden:

- Datentyp String
  Mit der Distanz zwischen zwei Strings wird im Allgemeinen die Unähnlichkeit der beiden String ermittelt. D.h. die Distanz ist umso geringer, je ähnlicher sich die beiden String sind. Beispielsweise kann die Distanz zwischen zwei Strings anhand der minimalen Anzahl von Einfüge-, Lösch- und Ersetz-Operationen berechnet werden, um den einen String in den anderen String umzuwandeln, was auch als Levenshtein-Distanz bekannt ist.

- Datentyp Zeit bzw. Zeitpunkt
  Die Distanz zwischen dem Zeitpunkt der einen Signatur und dem Zeitpunkt der anderen Signatur kann anhand des Zeitintervalls ermittelt werden, welches zwischen den beiden Zeitpunkten liegt. Das Zeitintervall kann in Millisekunden oder Tagen (oder jeder anderen Zeiteinheit) bestimmt werden, je nachdem welche Zeiteinheit die Zeitpunkte aufweisen.

- Datentyp Zahl
  Die Distanz zwischen zwei Attributen des Datentyps Zahl (z.B. Integer, Float) kann beispielsweise durch den Betrag ihrer Differenz ermittelt werden.

- Datentyp Vektor
  Die Distanz zwischen zwei Vektoren kann anhand der euklidischen Distanz gemäß der folgenden Gleichung (2) bestimmt werden.

$$d = \sqrt{\sum_{i=1}^{n}(x_i - y_i)^2} \qquad (2)$$

Dabei entspricht d der zu bestimmenden euklidischen Distanz, $x_i$ der i-ten Koordinate des Vektors X der einen Signatur und $y_i$ der i-ten Koordinate des Vektors Y der anderen Signatur und n der Anzahl der Dimensionen der beiden Vektoren.

- Datentyp Eigenschaftsmenge

Die Distanz zwischen zwei Eigenschaftsmengen kann beispielsweise anhand der übereinstimmenden Eigenschaften bestimmt werden. Je größer die Anzahl der übereinstimmenden Eigenschaften ist, desto kleiner ist die Distanz.

- Datentyp Hashwert
  Wenn zwei Attribute in Form von Hashwerten vorliegen, kann die Distanz 0 betragen, wenn die beiden Hashwerte identisch sind, und sonst 1 betragen.

- Datentyp Histogramm
  Wenn das Histogramm aus einer bestimmten Anzahl von Werten besteht, welche jeweils angeben, wie häufig ein bestimmter Pixelwert bei den Pixeln der Bildserie auftritt, kann der Betrag der Unterschiede dieser Werte für die beiden Signaturen bestimmt werden. Darüber hinaus kann die Distanz zwischen zwei Histogrammen gemäß der folgenden aus der Literatur bekannten Verfahren ermittelt werden: Chi-Sequare Distance, Lullback-Leibler Divergence, Wasserstein Metric.

- Datentyp Fourierspektrum
  Stellt man sich ein Fourierspektrum als ein zweidimensionales Bild dar, können die Pixelwerte dieser beiden Bilder der beiden Signaturen pixelweise subtrahiert werden, was auch als euklidische Distanz bekannt ist.
  Die vorab stehenden explizit aufgeführten Datentypen sind beispielhaft zu verstehen. Erfindungsgemäß können die Attribute der Signaturen auch weitere Datentypen aufweisen, wobei die Distanz bzw. Funktion DistAttr() zwischen diesen Attributen gemäß bekannter weiterer elementarer Distanzmetriken bestimmt werden kann.

Falls ein Attribut bei der einen Signatur nicht gesetzt ist, während das entsprechende Attribut bei der anderen Signatur gesetzt ist, entspricht die Distanz bzw. der Funktionswert der Funktion DistAttr() für diese Attribute dem maximalen Wert für eine Distanz zwischen einem beliebigen Wert für das Attribut bei der einen Signatur und einem beliebigen Wert für das Attribut bei der anderen Signatur. Dadurch ist sichergestellt, dass die Distanz zwischen zwei gesetzten Attributwerten nie größer als die Distanz zwischen einem gesetzten Attributwert und einem ungesetzten Attributwert sein kann.

Die Menge der Attribute, aus denen eine Signatur aufgebaut wird, umfasst insbesondere die so genannten DICOM-Attribute ("Digital Imaging and Communications in Medicine") der Bildserie (und ggfls. der Bilder) und kann zumindest eines von folgenden Attributen umfassen:

- Eine Beschreibung der zugehörigen Bildserie. Diese Beschreibung der Bildserie kann beispielsweise der (insbesondere eindeutigen) Bezeichnung der Bildserie entsprechen. Es ist aber auch möglich, dass die Beschreibung eine Menge von Schlagworten umfasst, welche bestimmte Eigenschaften der Bildserie beschreiben.

- Scanprotokoll-Parameter einer Bildgebungssequenz, mit welcher die zugehörige Bildserie erstellt werden soll. Dabei definieren die Scanprotokoll-Parameter (z.B. Repetitionszeit, Anzahl der Bildpunkte) die Art und Weise, wie die Daten zur Erzeugung der Bildserie von dem Untersuchungsobjekt erfasst werden.

- Werte, welche aus den Pixelwerten oder Pixeldaten, die anhand der erfassten Daten rekonstruiert worden sind, extrahiert werden können. Zu diesen Werten gehören beispielsweise Folgende:

  ◦ Ein Histogramm der zugehörigen Bildserie. Dabei besteht das Histogramm aus einer bestimmten Anzahl von Werten, die jeweils angeben, wie häufig ein bestimmter Pixelwert bei den Pixeln der Bildserie auftritt.
  ◦ Ein Fourier-Spektrum der zugehörigen Bildserie.

- Eine Information über anatomische Landmarks oder Charakteristika, welche in der zugehörigen Bildserie sichtbar sind. Beispielsweise kann mittels der Information darüber informiert werden, welche Organe, Knochen, Blutgefäße oder auch Läsionen in der jeweiligen Bildserie sichtbar sind.

Weitere Möglichkeiten für Attribute einer Signatur sind: der Bildtyp, die Modalität, der Institutsname, die Serienbeschreibung, Eigenschaften des Volumenabschnitts, von welchem die Bilder erzeugt werden, Erfassungskontrast, Protokollname, Herzfrequenz-Synchronisierungstechnik, Anzahl der Zeilen des Bildes, Anzahl der Spalten des Bildes, Anzahl der Bilder der Bildserie, eine zur Erstellung der Bildserie durchgeführte Kontrastmittelgabe usw.

Die jeweilige Signatur kann zum einen bestimmte Attribute der zugehörigen Bildserie umfassen. Es ist aber auch möglich, dass die Attribute der Signatur aus den Attributen der zugehörigen Bildserie abgeleitet werden. Wenn zumindest ein Teil der Attribute der Signatur aus den Attributen der Bildserie abgeleitet werden, ist die entsprechende Ableitungsregel insbesondere deterministisch und reproduzierbar.

Beispiele für die für die Ableitung von Attributen der Signatur aus den Attributen einer Bildserie sind die bereits vorab beschriebenen Histogramme oder Fourierspektren. Im Folgenden werden weitere Beispiele für die Ableitung von Attributen der Signatur aus den Attributen einer Bildserie beschrieben.

- Aus den beiden Attributen Startzeitpunkt der Datenerfassung und Endzeitpunkt der Datenerfassung der Bildserie kann die Differenz berechnet werden, um als Attribut der Signatur die Zeitdauer der Datenerfassung zu berechnen.
- Anstelle eines langen Strings mit einer kompletten Institutsadresse kann als Attribut der Signatur nur der Hashwert dieses Strings abgelegt sein.

Erfindungsgemäß ist es möglich, dass die jeweilige Signatur in einer (verlustfreien oder verlustbehafteten) komprimierten Form oder teilweise in Form von Hashwerten abgelegt wird.

Gemäß einer erfindungsgemäßen Ausführungsform wird ein Ähnlichkeitsmaß für eine Ähnlichkeit zwischen der Signaturmenge des jeweiligen Segments und derjenigen Signatur bestimmt, welche als ähnlichste Signatur ermittelt wird. Dieses Ähnlichkeitsmaß (oder Distanz) bestimmt dabei ein Maß für eine Ähnlichkeit zwischen der Signatur und der jeweiligen Signaturmenge. Für das jeweilige Segment wird nur dann die entsprechende Bildserie dargestellt, wenn das Ähnlichkeitsmaß, welches die Ähnlichkeit zwischen der Signatur und der Signaturmenge des Segments angibt, oberhalb einer vorbestimmten Ähnlichkeitsschwelle liegt. Diese Ähnlichkeitsschwelle kann dabei individuell pro Segment vorgegeben werden, so dass jedes Segment eine eigene Ähnlichkeitsschwelle aufweisen kann. In gleicher Weise kann pro Segment festgelegt werden, ob die jeweilige Ähnlichkeitsschwelle oder eine globale Ähnlichkeitsschwelle berücksichtigt werden soll oder ob jeweils die ähnlichste Bildserie dargestellt werden soll, auch wenn das Ähnlichkeitsmaß für die jeweilige Signatur nicht oberhalb der Ähnlichkeitsschwelle liegt.

Durch diese Ausführungsform kann vorteilhafterweise gewährleistet werden, dass die in einem Segment erfindungsgemäß dargestellte Bildserie eine bestimmte Ähnlichkeit mit der für das Segment vorgesehenen Bildserie aufweist. Falls keine Bildserie existiert, bei der die zugehörige Signatur eine Ähnlichkeit zu der Signaturmenge des Segments aufweist, die oberhalb der erforderlichen Ähnlichkeitsschwelle liegt, ist es bisweilen besser, keine Bildserie und kein Bild in dem Segment darzustellen, bevor eine falsche oder unpassende Bildserie oder ein falsches oder unpassendes Bild in einem Segment dargestellt wird.

Erfindungsgemäß ist es ebenfalls möglich, für eines oder für mehrere der Segmente jeweils eine Signatur aus der Menge der Signaturen der Bildserien zu ermitteln, welche zu der Signaturmenge des jeweiligen Segments am zweitähnlichsten ist. Entsprechend wird dann in dem jeweiligen Segment diejenige Bildserie dargestellt, deren Signatur als Zweitähnlichste ermittelt wurde.

Die vorab beschriebene Variante ermöglicht es beispielsweise dem auswertenden Arzt, die Auswertung der Bildserie eines bestimmten Segments anhand einer weiteren Bildserie dieses bestimmten Segments zu kontrollieren. Während die Bildserie des bestimmten Segments die ähnlichste Signatur aufweist, weist die weitere Bildserie des bestimmten Segments die zweitähnlichste Signatur auf.

Darüber hinaus ist es erfindungsgemäß auch möglich, noch weitere ähnliche Bildserien, z.B. die drittähnlichste, viertähnlichste usw. Bildserie im jeweiligen Segment darzustellen.

Vorteilhafterweise kann die Erstellung der Signatur für die jeweilige Bildserie bereits beim Erzeugen der Bildserie durch das bildgebende System durchgeführt werden.

Indem die Signatur bereits bei der Erzeugung der Bildserie erzeugt wird, wird vorteilhafterweise Berechnungszeit auf dem Befundungssystem selbst reduziert.

Gemäß einer weiteren bevorzugten erfindungsgemäßen Ausführungsform wird dieselbe Bildserie nur in einem der Segmente dargestellt.

Diese Ausführungsform verhindert vorteilhafterweise, dass dieselbe Bildserie in mehreren Segmenten dargestellt wird, da ihre Signatur (zufälligerweise) beispielsweise sowohl bezüglich der Signaturmenge des ersten Segments als auch bezüglich der Signaturmenge des zweiten Segments als ähnlichste Signatur bestimmt wird. In diesem Fall kann in dem entsprechenden Segment entweder keine Bildserie oder die nächst ähnliche Bildserie dargestellt werden. Mit anderen Worten sorgt diese Ausführungsform vorteilhafterweise dafür, dass in jedem Segment eine andere Bildserie dargestellt wird.

Im Rahmen der vorliegenden Erfindung wird auch ein bildgebendes System zur Erstellung mehrerer Bildserien und zum Darstellen von Bildern zur Auswertung durch eine Bedienperson bereitgestellt. Dabei umfasst das bildgebende System Mittel, um eine Bildserie eines Volumenabschnitts eines Untersuchungsobjekts zu erstellen, und eine Anzeige. Das bildgebende System ist ausgestaltet, um ein Layout mit mehreren Segmenten (oder mehrere Layouts mit jeweils mehreren Segmenten) zur Darstellung auf der Anzeige zu erzeugen, um eine jeweilige Signaturmenge an jedes Segment zuzuweisen, um den mehreren Bildserien jeweils eine Signatur zuzuweisen, um eine Signatur aus den Signaturen der Bildserien, welche zu der Signaturmenge des jeweiligen Segments am ähnlichsten ist, für jedes der Segmente zu ermitteln und um diejenige der Bildserien in dem jeweiligen Segment darzustellen, deren Signatur in dem vorherigen Schritt als ähnlichste ermittelt wurde.

Bei dem bildgebenden System kann es sich um ein System zur radiologischen Auswertung handeln, was erfindungsgemäß eine Magnetresonanzanlage, einen Computertomographen, eine Röntgenanlage, ein Tomosynthesegerät, eine Partikelbestrahlungsanlage, einen Positronen-Emissions-Tomographen, einen Einzelphotonen-Emissionscomputertomographen, einen PET-CT (Kombination aus einem Positronen-Emissions-Tomographen und einem Computertomographen) und einen mMR (Kombination aus einem Positronen-Emissions-Tomographen und einem Magnetresonanz-Tomographen) einschließt.

Die Vorteile des erfindungsgemäßen bildgebenden Systems entsprechen dabei den Vorteilen des erfindungsgemäßen Verfahrens zum Bestimmen von mittels eines bildgebenden Systems erzeugten Bildern zur Auswertung durch eine Bedienperson, welche vorab im Detail ausgeführt worden sind, so dass hier auf eine Wiederholung verzichtet wird.

Des Weiteren beschreibt die vorliegende Erfindung ein Computerprogrammprodukt, insbesondere ein Computerprogramm oder eine Software, welche man in einen Speicher einer programmierbaren Steuerung bzw. einer Recheneinheit eines bildgebenden Systems laden kann. Mit diesem Computerprogrammprodukt können alle oder verschiedene vorab beschriebene Ausführungsformen des erfindungsgemäßen Verfahrens ausgeführt werden, wenn das Computerprogrammprodukt in der Steuerung oder Steuereinrichtung des bildgebenden Systems läuft. Dabei benötigt das Computerprogrammprodukt eventuell Programmmittel, z.B. Bibliotheken und Hilfsfunktionen, um die entsprechenden Ausführungsformen der Verfahren zu realisieren. Mit anderen Worten soll mit dem auf das Computerprogrammprodukt gerichteten Anspruch insbesondere ein Computerprogramm oder eine Software unter Schutz gestellt werden, mit welcher eine der oben beschriebenen Ausführungsformen des erfindungsgemäßen Verfahrens ausgeführt werden kann bzw. welche diese Ausführungsform ausführt. Dabei kann es sich bei der Software um einen Quellcode (z.B. C++), der noch compiliert (übersetzt) und gebunden oder der nur interpretiert werden muss, oder um einen ausführbaren Softwarecode handeln, der zur Ausführung nur noch in die entsprechende Recheneinheit bzw. Steuereinrichtung zu laden ist.

Schließlich offenbart die vorliegende Erfindung einen elektronisch lesbaren Datenträger, z.B. eine DVD, ein Magnetband, eine Festplatte oder einen USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuereinrichtung bzw. Recheneinheit eines bildgebenden Systems gespeichert werden, können alle erfindungsgemäßen Ausführungsformen des vorab beschriebenen Verfahrens durchgeführt werden.

Die vorliegende Erfindung ermöglicht, dass eine Workflowbasierte Befundung auch mit kundenspezifischen Layouts ohne großen Konfigurationsaufwand eingesetzt werden kann.

Auch wenn sich die Messprogramme eines Kunden stark von den Standard-Messprogrammen eines Herstellers unterscheiden, kann erfindungsgemäß das Hersteller-konfigurierte Layout sehr schnell an die kundenspezifischen Messprogramme angepasst werden. Durch die vorliegende Erfindung kann die typische Konfigurationsphase, während welcher die Bildsegmente aller Layouts auf die Kunden-Messprogramme konfiguriert werden müssen, drastisch verkürzt werden.

Im Folgenden wird die vorliegende Erfindung anhand bevorzugter erfindungsgemäßer Ausführungsformen mit Bezug zu den Figuren im Detail beschrieben.

In Fig. 1 ist schematisch ein erfindungsgemäßes bildgebendes System dargestellt.

In Fig. 2 ist schematisch ein erfindungsgemäßes Vorgehen zum Bestimmen und Darstellen von Bildserien in einem Layout dargestellt.

Fig. 3 stellt ein Flussablaufdiagramm eines erfindungsgemäßen Verfahrens dar.

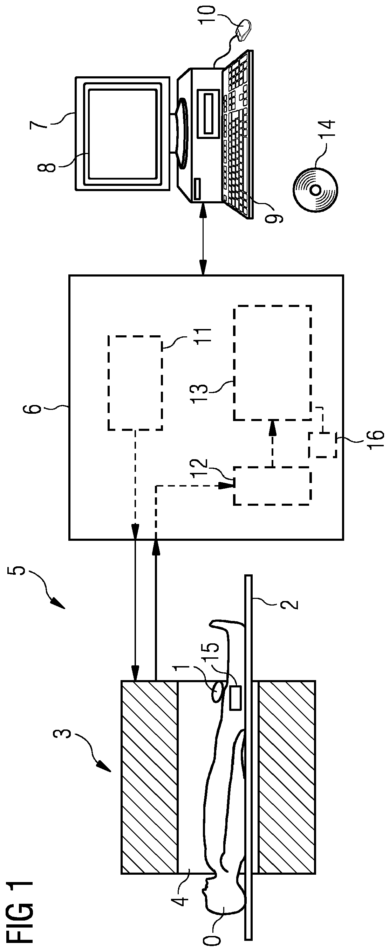

In Fig. 1 ist als Beispiel eines erfindungsgemäßen bildgebenden Systems schematisch eine Magnetresonanzanlage 5 dargestellt. Die Magnetresonanzanlage 5 umfasst im Wesentlichen einen Tomograph 3, mit welchem das für die MR-Untersuchung notwendige Magnetfeld in einem Messraum 4 erzeugt wird, einen Tisch oder Liegenbrett 2, eine Steuereinrichtung 6, mit welcher der Tomograph 3 gesteuert wird und MR-Daten von dem Tomograph 3 erfasst werden, und ein an die Steuereinrichtung 6 angeschlossenes Terminal 7.

Die Steuereinrichtung 6 umfasst ihrerseits eine Ansteuereinheit 11, eine Empfangsvorrichtung 12, eine Auswertevorrichtung 13 und einen Speicher 16. Während der Erstellung eines Bilddatensatzes werden MR-Daten mittels des Tomograph 3 von der Empfangsvorrichtung 12 erfasst, wobei der Tomograph 3 und der Tisch 2 von der Ansteuereinheit 11 derart angesteuert werden, dass MR-Daten in einem Messvolumen 15, welches sich im Körperinneren eines auf dem Tisch 2 liegenden Patienten O befindet, erfasst werden. Zur Erstellung einer Signatur einer Bildserie können die Attribute der Bildserie von der Steuereinrichtung 6 in den Speicher 16 geladen werden, um dann abhängig von den Attributen der Bildserie das Attribut oder die Attribute der Signatur zu bilden.

Die Auswertevorrichtung 13 bereitet dann die MR-Daten derart auf, dass sie auf einem Bildschirm 8 des Terminals 7 beispielsweise in einem Segment eines Layouts grafisch dargestellt werden können. Neben der grafischen Darstellung von Bildserien kann mit dem Terminal 7, welches neben dem Bildschirm 8 eine Tastatur 9 und eine Maus 10 umfasst, auch die Konfiguration eines Layouts vorgenommen werden. Über das Terminal 7 kann die Software für die Steuereinrichtung 6 in die Steuereinrichtung 6 geladen werden. Diese Software der Steuereinrichtung 6 kann dabei auch das erfindungsgemäße Verfahren umfassen. Es ist dabei auch möglich, dass ein erfindungsgemäßes Verfahren in einer Software enthalten ist, welche in dem Terminal 7 abläuft. Unabhängig davon, in welcher Software das erfindungsgemäße Verfahren enthalten ist, kann die Software auf einer DVD 14 gespeichert sein, so dass diese Software dann von dem Terminal 7 von der DVD 14 gelesen und entweder in die Steuereinrichtung 6 oder in eine Recheneinheit des Terminals 7 selbst kopiert werden kann.

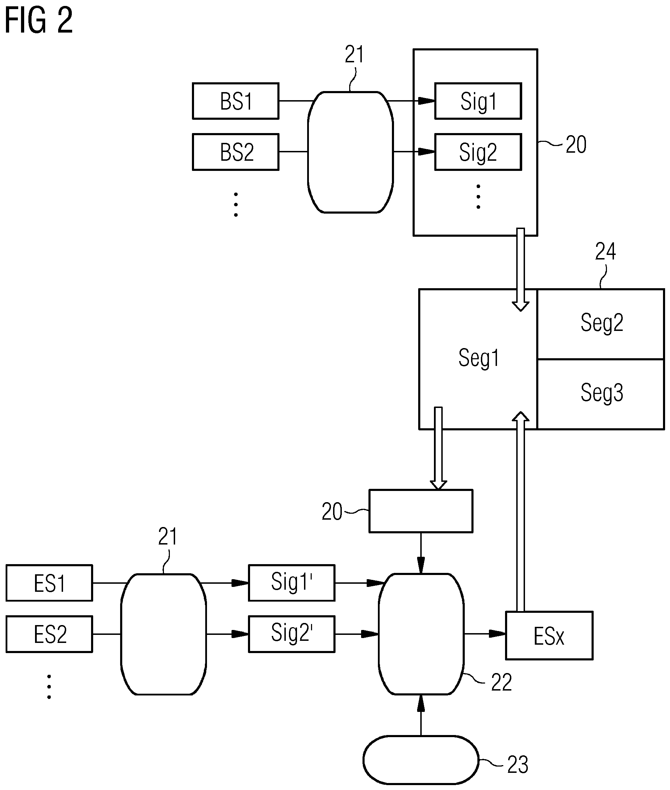

In Fig. 2 ist das erfindungsgemäße Vorgehen zur Konfiguration eines Layouts 24 schematisch dargestellt.

Beispielsweise kann in einem ersten Schritt pro Segment Seg1-3 des Layouts 24 eine Signaturmenge oder Signatursammlung 20 erstellt werden. Dieser Konfigurationsvorgang kann entweder beim Hersteller oder beim Kunden oder auch beim Hersteller und beim Kunden durchgeführt werden. Dazu werden pro Segment beispielhafte Bildserien BS1, BS2 erzeugt. Mit Hilfe eines Signatur-Extraktors 21 wird dann für jede dieser Bildserien BS1, BS2 eine Signatur Sig1, Sig2 erzeugt, welche in der zugehörigen Signaturmenge 20 abgelegt wird. Die jeweilige Signaturmenge 20 wird dann dem entsprechenden Segment Seg1-3 des Layouts 24 zugeordnet.

Das Layout 24 wird in der Regel beim Hersteller konfiguriert, d.h. in entsprechende Segmente Seg1-3 unterteilt, und an den Kunden ausgeliefert. Jedem Segment Seg1-3 ist eine entsprechende Signaturmenge 20 zugeordnet, wie es vorab beschrieben ist.

Zur Untersuchung eines bestimmten Untersuchungsobjekts O werden mit dem entsprechenden bildgebenden System Bildserien ES1, ES2 von dem Untersuchungsobjekt O erzeugt. Diese Bildserien werden zur Unterscheidung der vorab beschriebenen beispielhaften Bildserien BS1, BS2 im Folgenden als Eingangs-Bildserien ES1, ES2 bezeichnet. Für jede dieser Eingangs-Bildserien ES1, ES2 wird mit dem Signatur-Extraktor 21 eine Signatur Sig1', Sig2' erzeugt.

Um nun aus diesen zahlreichen Eingangs-Bildserien ES1, ES2 die jeweils passendste Bildserie zu bestimmen, welche in einem der Segmente Seg1-3 dargestellt wird, wird jede Signatur Sig1', Sig2' mittels einer Distanz-Metrik 23 mit der Signaturmenge 20 des jeweiligen Segments Seg1-3 in einer Vergleichsoperation 22 verglichen. Für diejenige Signatur Sig1', Sig2', welche die geringste Distanz zu der Signaturmenge 20 aufweist, wird die zugehörige Eingangs-Bildserie ESx ermittelt, die dann als die passendste Bildserie in dem entsprechenden Segment Seg1-3 dargestellt wird. Dieser Vorgang wird für alle Segmente Seg1-3 des Layouts 24 wiederholt.

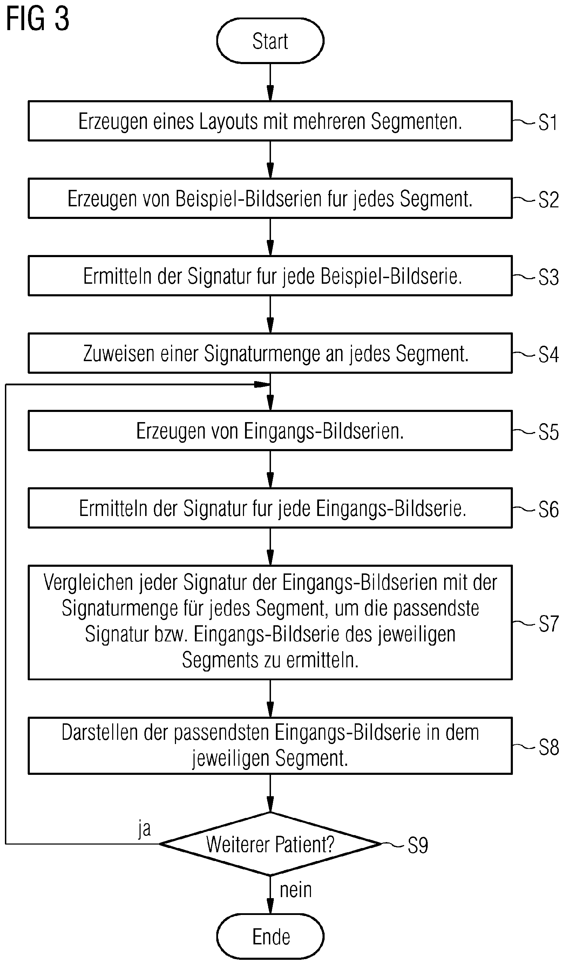

In Fig. 3 ist der Flussplan eines erfindungsgemäßen Verfahrens dargestellt.

Im Schritt S1 wird ein Layout 24 erzeugt, welches mehrere Segmente Seg1-3 umfasst. Im Schritt S2 werden für jedes Segment Seg1-3 eine oder mehrere Beispiel-Bildserien BS1, BS2 erzeugt, wobei für jede dieser Beispiel-Bildserien BS1, BS2 im folgenden Schritt S3 jeweils eine Signatur Sig1, Sig2 ermittelt wird. Im Schritt S4 wird jeweils eine Signaturmenge 20 an jedes der Segmente Seg1-3 zugewiesen. Dabei umfasst die jeweilige Signaturmenge 20 eines Segments Seg1-3 diejenigen Signaturen Sig1, Sig2, welche für diejenigen Beispiel-Bildserien BS1, BS2 erzeugt wurden, die für das jeweilige Segment Seg1-3 erstellt wurden.

Zur Untersuchung eines bestimmten Patienten werden im Schritt S5 mittels eines bildgebenden Systems (beispielsweise mittels einer Magnetresonanzanlage 5) mehrere Eingangs-Bildserien ES1, ES2 erzeugt. Es ist allerdings erfindungsgemäß auch möglich, dass von einem anderen System erzeugte Bildserien in ein Befundungssystem (z.B. PACS, syngo.via) geladen werden, um den bestimmten Patienten zu untersuchen. Für jede dieser Eingangs-Bildserien ES1, ES2 wird nun im Schritt S6 eine Signatur Sig1', Sig2' ermittelt. Im Schritt S7 wird ausgehend von den Signaturen Sig1', Sig2' der Eingangs-Bildserien ES1, ES2 diejenige Signatur ermittelt, welche die kleinste Distanz zu der Signaturmenge 20 des jeweiligen Segments Seg1-3 aufweist. Ausgehend von der derart ermittelten Signatur wird dann die zugehörige Eingangs-Bildserie ESx ermittelt, welche in dem jeweiligen Segment Seg1-3 im Schritt S8 dargestellt wird.

Wenn die Abfrage im Schritt S9 bejaht wird, wird ein weiterer Patient untersucht, so dass das erfindungsgemäße Verfahren zum Schritt S5 zurückkehrt. Wenn dagegen die Abfrage im Schritt S9 verneint wird, endet das erfindungsgemäße Verfahren.

Es ist ebenfalls möglich, dass zur Befundung eines Patienten mehrere Layouts (Befundungsschritte) durchlaufen werden. Darüber hinaus können mehrere Auswerte- und Mess-Einrichtungen eingesetzt werden, bevor derselbe Patient zu Ende befundet ist.

**Patentansprüche**

1. Verfahren zum Bestimmen von mittels eines bildgebenden Systems (5) erzeugten Bildern zur Auswertung durch eine Bedienperson, wobei das Verfahren folgende Schritte umfasst:

   Erzeugen eines Layouts (24) mit mehreren Segmenten (Seg1-3) zur Darstellung auf einer Anzeige (8), wobei in jedem der mehreren Segmente (Seg1-3) des Layouts (24) zumindest eines der erzeugten Bilder angezeigt werden soll,
   Erstellen jeweils einer Signatur (Sig1'; Sig2') für jede von mehreren Eingangs-Bildserien (ES1, ES2), indem aus einer Menge von Attributen der jeweiligen Eingangs-Bildserie (ES1; ES2) diese Signatur (Sig1'; Sig2') gebildet wird,

für jedes Segment werden folgende Schritte durchgeführt:

Zuweisen einer jeweiligen Signaturmenge (20) aus Signaturen von beispielhaften Bildserien (BS1, BS2) an jedes Segment (Seg1-3),

Ermitteln einer Signatur aus den Signaturen (Sig1', Sig2') der Eingangs-Bildserien (ES1, ES2), welche zu der jeweiligen Signaturmenge (20) des Segments (Seg1-3) am ähnlichsten ist,

wobei als die ähnlichste Signatur diejenige Signatur der Signaturen (Sig1', Sig2') der Eingangs-Bildserien (ES1, ES2) mit der geringsten Distanz zu den Signaturen der Signaturmenge bestimmt wird,

wobei die Distanz zwischen einer Signatur und einer Signaturmenge abhängig von Distanzen zwischen der Signatur und jeder Signatur der Signaturmenge bestimmt wird,

wobei jede Signatur (Sig1; Sig1'; Sig2; Sig2') dieselben Attribute umfasst,

wobei die Distanz zwischen der Signatur (Sig1'; Sig2') und jeder Signatur (Sig1; Sig2) der Signaturmenge (20) anhand einer Summe von Differenzen zwischen Attributwerten der Signatur (Sig1'; Sig2') und jeder Signatur (Sig1; Sig2) der Signaturmenge (20) bestimmt wird,

und

Darstellen derjenigen der Eingangs-Bildserien (ES1, ES2) in dem jeweiligen Segment (Seg1-3), deren Signatur in dem vorherigen Schritt als Ähnlichste ermittelt wurde, um dadurch die jeweils passendste zur Auswertung zu ermitteln,

wobei die Menge der Attribute, aus denen eine Signatur (Sig1; Sig2; Sig1'; Sig2') gebildet wird, zumindest eines von folgenden Attributen umfasst:

• ein DICOM-Attribut der zugehörigen Bildserie (BS1; BS2; ES1; ES2),
• Werte, welche aus Pixelwerten der zugehörigen Bildserie (BS1; BS2; ES1; ES2) extrahiert werden,
• ein Pixelwert-Histogramm der zugehörigen Bildserie (BS1; BS2; ES1; ES2),
• ein Fourier-Spektrum der zugehörigen Bildserie (BS1; BS2; ES1; ES2), und
• eine Information über anatomische Landmarks, welche in der zugehörigen Bildserie (BS1; BS2; ES1; ES2) sichtbar sind.

2. Verfahren nach Anspruch 1,
**dadurch gekennzeichnet,**
**dass** die Distanz zwischen einer Signatur (Sig1'; Sig2') und einer Signaturmenge (20) abhängig von Distanzen zwischen der Signatur (Sig1'; Sig2') und jeder Signatur (Sig1; Sig2) der Signaturmenge (20) bestimmt wird, indem die Distanz als Durchschnittswert dieser Distanzen bestimmt wird.

3. Verfahren nach Anspruch 1,
**dadurch gekennzeichnet,**
**dass** die Distanz zwischen einer Signatur (Sig1'; Sig2' und einer Signaturmenge (20) abhängig von Distanzen zwischen der Signatur (Sig1', Sig2') und jeder Signatur (Sig1; Sig2) der Signaturmenge (20) bestimmt wird, indem die Distanz als die gewichtete Summe dieser Distanzen bestimmt wird.

4. Verfahren nach einem der vorhergehenden Ansprüche,
**dadurch gekennzeichnet,**
**dass** der Schritt des Erstellens der Signatur (Sig1'; Sig2') ein Komprimieren der Signatur (Sig1'; Sig2') umfasst.

5. Verfahren nach einem der vorhergehenden Ansprüche,
**dadurch gekennzeichnet,**
**dass** das Verfahren zusätzlich umfasst:

Bestimmen eines Ähnlichkeitsmaßes, welches eine Ähnlichkeit zwischen einer Signatur (Sig1'; Sig2') und einer Signaturmenge (20) angibt, für eine Ähnlichkeit zwischen der Signaturmenge (20) des jeweiligen Segments (Seg1-3) und der Signatur (Sig1'; Sig2'), welche als Ähnlichste ermittelt wurde, und dass für das jeweilige Segment (Seg1-3) nur dann die Bildserie (ES1; ES2) dargestellt wird, wenn das bestimmte Ähnlichkeitsmaß eine vorbestimmte Ähnlichkeitsschwelle überschreitet.

6. Verfahren nach einem der vorhergehenden Ansprüche,
**dadurch gekennzeichnet,**
**dass** für eines der Segmente (Seg1-3) eine Signatur aus den Signaturen (Sig1', Sig2') der Bildserien (ES1, ES2) ermittelt wird, welche zu der Signaturmenge (20) des Segments (Seg1-3) am zweitähnlichsten ist, und
**dass** die Bildserie (ES1; ES2) in dem Segment (Seg1-3) dargestellt wird, deren Signatur (Sig1'; Sig2') als Zweitähnlichste ermittelt wurde.

7. Verfahren nach einem der vorhergehenden Ansprüche,
**dadurch gekennzeichnet,**
**dass** der Schritt des Erstellens der Signatur (Sig1; Sig2; Sig1'; Sig2') beim Erzeugen der Bildserie (BS1; BS2; ES1; ES2) durch das bildgebende System (5) ausgeführt wird.

8. Verfahren nach einem der vorhergehenden Ansprüche,
**dadurch gekennzeichnet,**
**dass** dieselbe der Eingangs-Bildserien (ES1; ES2) nur in einem der Segmente Seg1-3 dargestellt wird.

9. Bildgebendes System zur Erstellung mehrerer Eingangs-Bildserien (ES1; ES2) und zum Anzeigen von Bildern zur Auswertung durch eine Bedienperson,
wobei das bildgebende System (5) Mittel (3), um eine Eingangs-Bildserie (ES1; ES2) eines Volumenabschnitts (15) eines Untersuchungsobjekts (O) zu erstellen, eine Steuereinrichtung (6) und eine Anzeige (8) umfasst,
wobei das bildgebende System (5) ausgestaltet ist,
um ein Layout (24) mit mehreren Segmenten (Seg1-3) zur Darstellung auf der Anzeige (8) zu erzeugen,
um ausgehend von einer Menge von Attributen einer jeweiligen der Eingangs-Bildserien (ES1; ES2) eine Signatur (Sig1'; Sig2') für jede der Eingangs-Bildserien (ES1, ES2) zu erstellen,
um eine jeweilige Signaturmenge (20) aus Signaturen von beispielhaften Bildserien (BS1, BS2) an jedes Segment (Seg1-3) zuzuweisen,
um aus den Signaturen (Sig1', Sig2') der Bildserien (ES1, ES2) eine Signatur zu ermitteln, welche zu der jeweiligen Signaturmenge (20) des Segments (Seg1-3) am ähnlichsten ist, um als die ähnlichste Signatur diejenige Signatur der Signaturen (Sig1', Sig2') der Eingangs-Bildserien (ES1, ES2) mit der geringsten Distanz zu den Signaturen der Signaturmenge zu bestimmen,
um die Distanz zwischen einer Signatur und einer Signaturmenge abhängig von Distanzen zwischen der Signatur und jeder Signatur der Signaturmenge zu bestimmen,
wobei jede Signatur (Sig1; Sig1'; Sig2; Sig2') dieselben Attribute umfasst,
um die Distanz zwischen der Signatur (Sig1'; Sig2') und jeder Signatur (Sig1; Sig2) der Signaturmenge (20) anhand einer Summe von Differenzen zwischen Attributwerten der Signatur (Sig1'; Sig2') und jeder Signatur (Sig1; Sig2) der Signaturmenge (20) zu bestimmen, und
um diejenigen der Eingangs-Bildserien (ES1, ES2) in dem jeweiligen Segment (Seg1-3) auf der Anzeige (8) darzustellen, deren Signatur vorher als Ähnlichste ermittelt wurde, um dadurch die jeweils passendste zur Auswertung zu ermitteln,
wobei die Menge der Attribute, aus denen eine Signatur (Sig1; Sig2; Sig1'; Sig2') gebildet wird, zumindest eines von folgenden Attributen umfasst:

- DICOM-Werte der zugehörigen Bildserie (BS1; BS2; ES1; ES2),
- Werte, welche aus Pixelwerten der zugehörigen Bildserie (BS1; BS2; ES1; ES2) extrahiert werden,
- ein Pixelwert-Histogramm der zugehörigen Bildserie (BS1; BS2; ES1; ES2),
- ein Fourier-Spektrum der zugehörigen Bildserie (BS1; BS2; ES1; ES2), und
- eine Information über anatomische Landmarks, welche in der zugehörigen Bildserie (BS1; BS2; ES1; ES2) sichtbar sind.

10. Bildgebendes System nach Anspruch 9,
**dadurch gekennzeichnet,**
**dass** das bildgebende System (5) zur Durchführung des Verfahrens nach einem der Ansprüche 2-8 ausgestaltet ist.

11. Computerprogrammprodukt, welches ein Programm umfasst und direkt in einen Speicher einer programmierbaren Steuereinrichtung (6) eines bildgebenden Systems (5) ladbar ist, mit Programm-Mitteln, um alle Schritte des Verfahrens nach einem der Ansprüche 1-8 auszuführen, wenn das Programm in der Steuereinrichtung (6) des bildgebenden Systems (5) ausgeführt wird.

12. Elektronisch lesbarer Datenträger mit darauf gespeicherten elektronisch lesbaren Steuerinformationen, welche derart ausgestaltet sind, dass sie bei Verwendung des Datenträgers (14) in einer Steuereinrichtung (6) eines bildgebenden Systems (5) das Verfahren nach einem der Ansprüche 1-8 durchführen.

**Claims**

1. Method for the determination of images created by means of an imaging system (5) for evaluation by an operator, wherein the method comprises the following steps:

creation of a layout (24) with a plurality of segments (Seg1-3) for depiction on a display (8), wherein in each of the plurality of segments (Seg1-3) of the layout (24) at least one of the images created is to be displayed, compilation in each case of a signature (Sig1'; Sig2') for each of a plurality of input image series (ES1, ES2) in that this signature (Sig1'; Sig2') is formed from a set of attributes of the respective input image series (ES1; ES2), the following steps are performed for each segment:

assignment of a respective signature set (20) consisting of signatures from exemplary image series (BS1, BS2) to each segment (Seg1-3), ascertainment of a signature from the signatures (Sig1'. Sig2') of the input image series (ES1, ES2), which is most similar to the respective signature set (20) of the segment (Seg1-3), wherein the most similar signature is determined to be the signature of the signatures (Sig1';, Sig2') of the input image series (ES1, ES2) with the shortest distance to the signatures of the signature set, wherein the distance between a signature and a signature set is determined in dependence on distances between the signature and each signature of the signature set, wherein each signature (Sig1; Sig1'; Sig2; Sig2') has the same attributes, wherein the distance between the signature (Sig1'; Sig2') and each signature (Sig1; Sig2) of the signature set (20) is determined with reference to a sum of differences between attribute values of the signature (Sig1'; Sig2') and each signature (Sig1; Sig2) of the signature set (20), and depiction of the input image series (ES1, ES2) in the respective segment (Seg1-3) whose signature was ascertained in the preceding step as the most similar signature, in order to determine the most appropriate for evaluation in each case as a result, wherein the set of attributes from which a signature (Sig1; Sig2; Sig1'; Sig2') is formed comprises at least one of the following attributes:

• a DICOM attribute of the associated image series (BS1; BS2; ES1; ES2),
• values, which are extracted from pixel values of the associated image series (BS1; BS2; ES1; ES2),
• a pixel-value histogram of the associated image series (BS1; BS2; ES1; ES2),
• a Fourier spectrum of the associated image series (BS1; BS2; ES1; ES2) and
• information on anatomical landmarks, which are visible in the associated image series (BS1; BS2; ES1; ES2).

2. Method according to claim 1,
**characterised in that**
the distance between a signature (Sig1'; Sig2') and a signature set (20) is determined in dependence on distances between the signature (Sig1'; Sig2') and each signature (Sig1; Sig2) of the signature set (20) **in that** the distance is determined as the average value of these distances.

3. Method according to claim 1,
**characterised in that**
the distance between a signature (Sig1'; Sig2') and a signature set (20) is determined in dependence on distances between the signature (Sig1', Sig2') and each signature (Sig1; Sig2) of the signature set (20) **in that** the distance is determined as the weighted sum of these distances.

4. Method according to one of the preceding claims,
**characterised in that**
the step of the compilation of the signature (Sig1'; Sig2') comprises the compression of the signature (Sig1'; Sig2').

5. Method according to one of the preceding claims,
**characterised in that**

the method additionally comprises:

determination of a degree of similarity indicating the similarity between a signature (Sig1'; Sig2') and a signature set (20) for a similarity between the signature set (20) of the respective segment (Seg1-3) and the signature (Sig1'; Sig2') that was ascertained as most similar and that the image series (ES1; ES2) for the respective segment (Seg1-3) is only depicted when the determined degree of similarity exceeds a prespecified similarity threshold.

6. Method according to one of the preceding claims,
   **characterised in that**
   for one of the segments (Seg1-3) a signature is ascertained from the signatures (Sig1', Sig2') of the image series (ES1, ES2) that is second most similar to the signature set (20) of the segment (Seg1-3), and
   that the image series (ES1; ES2) displayed in the segment (Seg1-3) is that whose signature (Sig1'; Sig2') was ascertained as second most similar.

7. Method according to one of the preceding claims,
   **characterised in that**
   the step of the compilation of the signature (Sig1; Sig2; Sig1'; Sig2') is performed during the creation of the image series (BS1; BS2; ES1; ES2) by the imaging system (5).

8. Method according to one of the preceding claims,
   **characterised in that**
   the same one of the image series (ES1; ES2) is only depicted in one of the segments (Seg1-3).

9. Imaging system for compiling a plurality of image series (ES1; ES2) and for displaying images for evaluation by an operator,
   wherein the imaging system (5) comprises means (3) for compiling an input image series (ES1; ES2) of a volume segment (15) of an examination object (O), a control facility (6) and a display (8),
   wherein the imaging system (5) is embodied
   to create a layout (24) with a plurality of segments (Seg1-3) for depiction on the display (8),
   to compile a signature (Sig1'; Sig2') for each of the image series (ES1, ES2) starting from a set of attributes of a respective one of the input image series (ES1; ES2),
   to assign a respective signature set (20) consisting of signatures of exemplary image series (BS1 BS2) to each segment (Seg1-3),
   to ascertain a signature from the signatures (Sig1'; Sig2') of the image series (ES1, ES2) that is most similar to the respective signature set (20) of the segment (Seg1-3), to determine as the most similar signature the signature of the signatures (Sig1', Sig2') of the input image series (ES1, ES2) with the shortest distance to the signatures of the signature set,
   to determine the distance between a signature and a signature set in dependence on distances between the signature and each signature of the signature set,
   wherein each signature (Sig1; Sig1'; Sig2; Sig2') has the same attributes,
   to determine the distance between the signature (Sig1'; Sig2') and each signature (Sig1; Sig2) of the signature set (20) with reference to a sum of differences between attribute values of the signature (Sig1'; Sig2') and each signature (Sig1; Sig2) of the signature set (20),
   and
   to depict on the display (8) the input image series (ES1, ES2) in the respective segment (Seg1-3) whose signature was previously ascertained as the most similar signature, in order to determine the most appropriate for evaluation in each case as a result,
   wherein the set of attributes from which a signature (Sig1; Sig2; Sig1'; Sig2') is formed comprises at least one of the following attributes:

   • a DICOM attribute of the associated image series (BS1; BS2; ES1; ES2),
   • values, which are extracted from pixel values of the associated image series (BS1; BS2; ES1; ES2),
   • a pixel-value histogram of the associated image series (BS1; BS2; ES1; ES2),
   • a Fourier spectrum of the associated image series (BS1; BS2; ES1; ES2) and
   • information on anatomical landmarks, which are visible in the associated image series (BS1; BS2; ES1; ES2).

10. Imaging system according to claim 9,

**characterised in that**
the imaging system (5) is embodied to carry out the method according to one of claims 2-8.

11. Computer program product which comprises a program and can be loaded directly into a memory of a programmable control facility (6) of an imaging system (5) with program means for carrying out all the steps of the method according to one of claims 1-8 when the program is executed in the control facility (6) of the imaging system (5).

12. Electronically readable data carrier with electronically readable control information stored thereupon, which is embodied to carry out the method according to one of claims 1-8 when the data carrier (14) is used in a control facility (6) of an imaging system (5).

**Revendications**

1. Procédé de détermination d'images produites au moyen d'un système (5) d'imagerie pour l'interprétation par une personne de service, le procédé comprenant les stades suivants :

production d'un layout (24) ayant plusieurs segments (Seg1 à 3) pour la représentation sur un affichage (8), dans lequel, dans chacun des plusieurs segments (Seg1 à 3) du layout (24) au moins l'une des images produites doit être affichée,
établissement respectivement d'une signature (Sig1'; Sig2') pour chacune de plusieurs séries (ES1, ES2) d'images d'entrée en formant cette signature (Sig1'; Sig2') à partir d'un ensemble d'attributs de la série (ES1, ES2) d'images d'entrée respectives,
pour chaque segment, on effectue les stades suivants :

affectation à chaque segment (Seg1 à 3) d'un ensemble (20) respectif de signatures parmi les signatures de série (BS1, BS2) à titre d'exemple,
détermination d'une signature parmi les signatures (Sig1'; Sig2') des séries (ES1, ES2) d'images d'entrée, qui est la plus semblable à l'ensemble (20) de signatures respectif du segment (Seg 1 à 3),
dans lequel on détermine, comme la signature la plus semblable, la signature parmi les signatures (Sig1'; Sig2') des séries (ES1, ES2) d'images d'entrée ayant la distance la plus petite aux signatures de l'ensemble de signatures,
dans lequel on détermine la distance entre une signature et un ensemble de signatures en fonction de distances entre la signature et chaque signature de l'ensemble de signatures,
dans lequel chaque signature (Sig1; Sig1'; Sig2; Sig2') comprend les mêmes attributs,
dans lequel on détermine la distance entre la signature (Sig1'; Sig2') et chaque signature (Sig1; Sig2) de l'ensemble (20) de signatures à l'aide d'une somme de différence entre des valeurs d'attribut de la signature (Sig1'; Sig2') et de chaque signature (Sig1; Sig2) de l'ensemble (20) de signatures, et
représentation des séries (ES1, ES2) d'images d'entrée dans le segment (Seg1 à 3) respectif, dont la signature a été déterminée comme la plus semblable dans le stade précédent, afin de déterminer ainsi celle qui convient le mieux pour l'interprétation,
dans lequel l'ensemble des attributs, à partir desquels une signature (Sig1; Sig1'; Sig2; Sig2') est formée, comprend au moins l'un des attributs suivants

• un attribut DICOM de la série (BS1; BS2; ES1, ES2) d'images associée,
• des valeurs extraites de valeurs de pixel de la série (BS1; BS2; ES1, ES2) d'images associée,
• un histogramme de valeurs de pixel de la série (BS1; BS2; ES1, ES2) d'images associée,
• un spectre de Fourier de la série (BS1; BS2; ES1, ES2) d'images associée et
• une information sur des jalons anatomiques visibles dans la série (BS1; BS2; ES1, ES2) d'images associée.

2. Procédé suivant la revendication 1,
**caractérisé**
**en ce que** l'on détermine la distance entre une signature (Sig1'; Sig2') et un ensemble (20) de signatures en fonction de distances entre la signature (Sig1'; Sig2') et chaque signature (Sig1; Sig2) de l'ensemble (20) de signatures, en déterminant la distance comme étant la valeur moyenne de ces distances.

**3.** Procédé suivant la revendication 1,
**caractérisé**
**en ce que** l'on détermine la distance entre une signature (Sig1'; Sig2') et un ensemble (20) de signatures en fonction de distances entre la signature (Sig1'; Sig2') et chaque signature (Sig1; Sig2) de l'ensemble (20) de signatures, en déterminant la distance comme étant la somme pondérée de ces distances.

**4.** Procédé suivant l'une des revendications précédentes,
**caractérisé**
**en ce que** le stade d'établissement de la signature (Sig1'; Sig2') comprend une compression de la signature (Sig1'; Sig2').

**5.** Procédé suivant l'une des revendications précédentes,
**caractérisé**
**en ce que** le procédé comprend, en outre :

la détermination d'un degré de similitude, qui indique une similitude entre une signature (Sig1'; Sig2') et un ensemble (20) de signatures, pour une similitude entre l'ensemble (20) de signatures du segment (Seg1 à 3) respectif et la signature (Sig1'; Sig2'), qui a été déterminée comme la plus semblable et en ce que l'on ne représente, pour le segment (Seg1 à 3) respectif, la série (ES1; ES3) d'images que si le degré de similitude déterminé dépasse un seuil de similitude déterminé à l'avance.

**6.** Procédé suivant l'une des revendications précédentes,
**caractérisé**
**en ce que**, pour l'un des segments (Seg 1 à 3), l'on détermine une signature parmi les signatures (Sig1'; Sig2') de séries (ES1; ES2) d'images, qui est la plus semblable en second à l'ensemble (20) de signatures du segment (Seg1 à 3) et
**en ce que** l'on représente la série (ES1; ES2) d'images dans le segment (Seg1 à 3), dont la signature (Sig1'; Sig2') a été déterminée comme la plus semblable en second.

**7.** Procédé suivant l'une des revendications précédentes,
**caractérisé**
**en ce que** l'on effectue, par le système (5) d'imagerie, le stade d'établissement de la signature (Sig1; Sig1'; Sig2; Sig2') lors de la production de la série (BS1; BS2; ES1; ES2) d'images.

**8.** Procédé suivant l'une des revendications précédentes,
**caractérisé**
**en ce que** l'on ne représente la même des séries (ES1; ES2) d'images d'entrée que dans l'un des segments (Seg1 à 3).

**9.** Système d'imagerie pour établir plusieurs séries (ES1; ES2) d'images d'entrée et pour afficher des images à interpréter par une personne de service,
dans lequel le système (5) d'imagerie comprend des moyens (3) pour établir une série (ES1; ES2) d'images d'entrée d'une partie (15) en volume d'un objet (O) à étudier, un dispositif (6) de commande et un affichage (8),
dans lequel le système (5) d'imagerie est conformé,
pour produire un layout (24) ayant plusieurs segments (Seg1 à 3) pour la représentation sur un affichage (8),
pour établir, à partir d'un ensemble d'attributs d'une série (ES1; ES2) d'images respective, une signature (Sig1'; Sig2') chacune des séries (ES1; ES2) d'images d'entrée, pour affecter à chaque segment (Seg1 à 3) un ensemble (20) de signatures respectif parmi les signatures de série (BS1; BS2) d'images à titre d'exemple,
pour déterminer, parmi les signatures (Sig1'; Sig2') des séries (ES1; ES2) d'images, une signature, qui est la plus semblable à l'ensemble (20) de signatures respectif du segment (Seg1 à 3),
pour déterminer, comme signature la plus semblable, la signature parmi les signatures (Sig1'; Sig2') des séries (ES1; ES2) d'images d'entrée ayant la distance la plus petite aux signatures de l'ensemble de signatures,
pour déterminer la distance entre une signature et un ensemble de signatures en fonction de distances entre la signature et chaque signature de l'ensemble de signatures,
chaque signature (Sig1; Sig1'; Sig2; Sig2') comprenant les mêmes attributs,
pour déterminer la distance entre la signature (Sig1'; Sig2') et chaque signature (Sig1; Sig2) de l'ensemble (20) de signatures à l'aide d'une somme de différence entre des valeurs d'attribut de la signature (Sig1'; Sig2') et de chaque signature (Sig1; Sig2) de l'ensemble (20) de signatures,

et

pour représenter les séries (ES1, ES2) d'images d'entrée dans le segment (Seg1 à 3) respectif sur l'affichage (8), dont la signature a été déterminée auparavant comme la plus semblable afin de déterminer ainsi celle qui convient le mieux pour l'interprétation,

l'ensemble des attributs, à partir desquels une signature (Sig1; Sig1'; Sig2; Sig2') est formée, comprend au moins l'un des attributs suivants :

- un attribut DICOM de la série (BS1; BS2; ES1, ES2) d'images associée,
- des valeurs extraites de valeurs de pixel de la série (BS1; BS2; ES1, ES2) d'images associée,
- un histogramme de valeurs de pixel de la série (BS1; BS2; ES1, ES2) d'images associée,
- un spectre de Fourier de la série (BS1; BS2; ES1, ES2) d'images associée et
- une information sur des jalons anatomiques visibles dans la série (BS1; BS2; ES1, ES2) d'images associée.

10. Système d'imagerie suivant la revendication 9,
**caractérisé**
**en ce que** le système (5) d'imagerie est conformé pour effectuer le procédé suivant l'une des revendications 2 à 8.

11. Produit de programme d'ordinateur, qui comprend un programme et qui peut être chargé directement dans une mémoire d'un dispositif (6) de commande programmable d'un système (5) d'imagerie, comprenant des moyens de programme pour exécuter tous les stades du procédé suivant l'une des revendications 1 à 8, lorsque le programme est réalisé dans le dispositif (6) de commande du système (5) d'imagerie.

12. Support de données déchiffrables électroniquement ayant des informations de commande déchiffrables électroniquement qui y sont mémorisées et qui sont conformées de manière à ce qu'elles effectuent, lors de l'utilisation du support (14) de données dans un dispositif (6) de commande d'un système (5) d'imagerie, le procédé suivant l'une des revendications 1 à 8.

FIG 1

EP 3 214 562 B1

## FIG 2

# FIG 3

Start

Erzeugen eines Layouts mit mehreren Segmenten. ~S1

Erzeugen von Beispiel-Bildserien fur jedes Segment. ~S2

Ermitteln der Signatur fur jede Beispiel-Bildserie. ~S3

Zuweisen einer Signaturmenge an jedes Segment. ~S4

Erzeugen von Eingangs-Bildserien. ~S5

Ermitteln der Signatur fur jede Eingangs-Bildserie. ~S6

Vergleichen jeder Signatur der Eingangs-Bildserien mit der Signaturmenge für jedes Segment, um die passendste Signatur bzw. Eingangs-Bildserie des jeweiligen Segments zu ermitteln. ~S7

Darstellen der passendsten Eingangs-Bildserie in dem jeweiligen Segment. ~S8

ja    Weiterer Patient? ~S9

nein

Ende

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20100232661 A1
- US 20150161147 A1
- US 20150154356 A1